# EUROPEAN PATENT SPECIFICATION

(11) **EP 1 476 426 B1**
(45) Date of publication and mention of the grant of the patent: **23.08.2006**
(21) Application number: 03742548.5
(22) Date of filing: 19.02.2003
(51) Int. Cl.: C07D 209/36, A61K 31/404, A61P 35/00, C07H 15/26

(54) **HYDROXYLATED INDIRUBIN DERIVATIVES**
HYDROXYLIERTE INDIRUBINDERIVATE
DERIVES DE L'INDIRUBINE HYDROXYLES

(30) Priority: 20.02.2002 EP 02003241
(43) Date of publication of application: 17.11.2004
(73) Proprietor: Eisenbrand, Gerhard, Prof. Dr., 69120 Heidelberg (DE)
(72) Inventor: EISENBRAND, Gerhard, 69120 Heidelberg (DE); HIPPE, Frankie, L-8221 Mamer (LU)
(74) Representative: Müller-Boré & Partner Patentanwälte
(86) International application number: PCT/EP2003/001679
(87) International publication number: WO 2003/070703

(56) References cited:
- WO-A-00/61124
- WO-A-00/61555

## Description

The present invention relates to hydroxylated indirubin derivatives which can be used for the manufacture of a medicament for the treatment of solid cancers and leukemia and metastases thereof.

Indigoid bisindoles comprise a spectrum of natural dyestuffs. Many of these can be obtained from plants. Accordingly, indirubin, indigo and isoindigo which are all indigoid bisindoles derivatives, are natural products which can be obtained from different plants: namely, Baphicacanthus cusia (Acanthaceae), Indigofera suffruticosa (Fabaceae), lsatis indigotica (Brassicaceae) and others. Indican, a glycoside which is found in plants, gives glucose and 3-hydroxyindole due to acidic or enzymatic hydrolysis. 3-Hydroxyindole is converted by air-oxidation into indigo and its isomers. Indigo naturalis (Chinese: Quing Dai) is the natural blue dye obtained from plant material, e.g. Isatis indigotica (Brassicaceae). Indirubin, an isomer of indigo, can be found in Indigo naturalis as a by-product (Falbe J. & Regitz M., Römpp Chemie Lexikon (1992), 9. Aufl., Stuttgart, Georg Thieme Verlag). It occurs also in lsatis tinctoria in an amount of up to 5% which is indigenous to Central Europe (Gelius R., Z. Chem., 20, (1980), 340-341). Derivatives of indirubin are known for a long time as dyes of low persistence.

Indigo naturalis is reported to be used in traditional Chinese medicine as a haemostatic, antipyretic, antiinflammatory and sedative agent in the treatment of bacterial and viral infections. Antileukemic effects of Indigo naturalis have also been reported, with indirubin being the effective principle (Ji X. et al., Acta Pharm. Sin., 16, (1981), 146-148; Gan W. J. et al., J. Hematol., 6, (1985), 611-613). In spite of its antileukaemic activity, however, indirubin dissolves only poorly in water and is therefore not readily resorbed. Recently, the antileukemic activity of some better soluble indirubin derivatives has been reported (Ch. Li et a., Bull. Chem. Soc. Jpn. 69, 1621-1627,(1996)). For indigoid bisindoles, in order to be used in the treatment of human solid tumors and leukemia one of the major topics is to provide compounds with improved bioavailability.

Thus, the technical problem underlying the present invention is to provide active substances which can be used in the treatment of human solid tumors and leukemia and metastases thereof. Furthermore, the absorbability of said substances should be improved in order to improve their bioavailability and their *in vivo* antitumor activity.

The solution to the above technical problem is achieved by the embodiments characterized in the claims.

In particular, the present invention relates to cell membrane penetrating indirubin derivatives having the following general formula 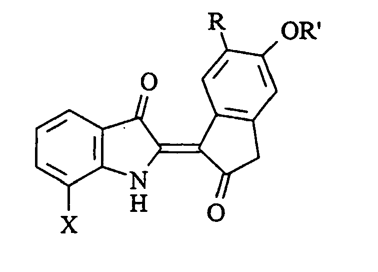 wherein the group R represents a straight-chain or branched-chain alkyl group or a straight-chain or branched-chain alkoxy group each having 1 to 18 carbon atoms, preferably 1 to 4 carbon atoms, the group R' represents H, a straight-chain or branched-chain alkyl having 1 to 4 carbon atoms or a glucuronide group, and X is H or OH. Particularly, the group R stands for methyl, ethyl, n-propyl, n-butyl, methoxy, ethoxy, propoxy and butoxy. Particularly, the group R' stands for H or a glucuronide group.

Preferred compounds of the indirubin derivatives of the present invention are 6-hydroxy-5-methylindirubin and 6,7'-dihydroxy-5-methylindirubin and their glucuronide derivatives, i.e. 3,4,5-trihydroxy-6-(5-methyl-1*H*,1'*H*-[2',3]bis-indolyliden-2,3'-dion-6-yl)-tetrahydropyran-2-carboxylic acid and 3,4,5-trihydroxy-6-(7'-hydroxy-5-methyl-1*H*,1'*H*-[2',3]bisindolyliden-2,3'-dion-6-yl)-tetrahydropyran-2-carboxylic acid, respectively.

5-Methylindirubin has been proven to be highly effective on human solid tumors implanted into nude mice without any perceptible toxicity. To the contrary, the parent compound indirubin is considerably less effective, but already remarkably toxic as evidenced by considerable body weight loss at a comparable dosage; cf. PCT/EP00/03210. It has now surprisingly been found that the incorporation of, for example, a methyl group or a methoxy group into the 5-position of indirubin renders the molecule easily metabolizable. This is in contrast to the unsubstituted parent compound indirubin which is not metabolized to a detectable extent under the same conditions. In particular, two main metabolites have been isolated and structurally determined, namely 6-hydroxy-5-methylindirubin and 6,7'-dihydroxy-5-methylindirubin. Both metabolites are at least as effective in inhibiting growth of tumor cells as the parent compounds. Therefore, oxidative metabolism in the liver obviously results in an enhancement of the antitumor activity and in increased solubility. Whereas in many cases metabolic oxidation results in decreased biological activity, in the present case the resulting metabolites are at least as active as the parent compounds. Thus, small variations in the substitution pattern of indirubin surprisingly gives rise to remarkable changes in the biological activity.

The antitumor active compounds according to the present invention can be used for the manufacture of a medicament for the treatment of human solid tumors and leukemia and metastases thereof. The term "human solid tumors" according to the present invention preferably includes carcinomas, melanomas, adenomas, sarcomas, lymphomas, neuroblastomas, teratomas and astrocytomas. Specific examples are mammary carcinomas, lung carcinomas, stomach and liver carcinomas, colon carcinomas, bladder carcinomas, ovarian carcinomas, pancreatic carcinomas, renal carcinomas, prostatic carcinomas, bronchial carcinomas, laryngeal carcinomas and the like.

In the pharmaceutical formulations according to the present invention, the indirubin derivatives can also be employed in the form of their physiologically acceptable salts. The above identified indirubin derivatives of the present invention can be formulated into pharmaceutical compositions which contain optionally a pharmaceutically acceptable carrier and/or diluent. Said pharmaceutical compositions can be applied e.g. orally, topically, intravenously, intraperitoneally, subcutaneously and rectally in pharmaceutically effective amounts.

One general problem in the field of pharmacology is the formulation of pharmaceutically active substances in pharmaceutical compositions which can be applied to a human body. Since most physiological fluids are water-based, the pharmaceutically active substances should be soluble in water and/or a water miscible solvent wherein the latter of course has to be physiologically acceptable in small concentrations, such as ethanol. Furthermore, pharmaceutically active substances to be applied orally have to be absorbed into the human body and have to permeate the gastrointestinal mucous membrane to be taken up into the portal blood circulation to reach the liver. By the same token, compounds applied parenterally, e.g. intravenously, need to cross several membranes in order to reach and to penetrate the target cells of the tumor.

Cellular membranes are composed of lipid bilayers, i.e. composed of a rather non-polar medium. Therefore, substitution with very polar groups on the one hand improves the water solubility of a compound but on the other hand hinders or even prohibits the absorption of antitumor active substances into a tumor cell. Thus, antitumor active substances which show good inhibitory activities under certain *in vitro* conditions towards proliferation related targets such as growth factor receptors, kinases and other signalling enzymes have to be rejected because of not showing any activity when tested using intact cells or organisms.

Therefore, the indirubin derivatives according to the present invention are cell membrane penetrating indirubines. According to the present invention the term "cell membrane penetrating" means the ability of the indirubin derivatives to be taken up by the tumor cell through the cellular membrane.

The chemical incorporation of an alkyl or alkoxy substituent into the 5-position of indirubin brings about a drastic change in biotransformation behaviour and makes 5-alkyl-substituted or 5-alkoxy-substituted inrubin derivatives easily accessible to oxidative metabolism. It has been found that this oxidative metabolism is mediated by cytochrom P450 dependent liver monooxygenases. Experiments in liver microsome preparations from cows, pigs and rats and also from a spectrum of human liver samples have shown that for example 5-methylindirubin is rapidly metabolized, yielding ring-hydroxylated indirubin derivatives as major metabolites besides small amounts of further metabolites.

Cyp450 isoenzymes in human liver microsomes found to be mainly responsible for metabolic hydroxylation, were Cyp1A2 and Cyp3A4, which together provide for more than 50% of the average cytochrome P450 activity in human liver. There are, however, considerable variations of expression levels and activities of these enzymes. This interindividual variability results from genetic polymorphisms on the one side and, on the other side, from the marked inhibitory as well as inducing effects on enzymatic activity brought about by certain food constituents or drugs. Thus, the highly variable situation in metabolizing activity of humans reflects the individual genetic make-up, as well as inductive/inhibitory effects of cosubstrates such as food constituents, drugs, etc. on cytochrome P450 activity. It is therefore a great advantage to minimize this primary source of variability that directly influences drug action and bioavailability, by utilizing the respective biologically active ring-hydroxylated metabolites themselves, i.e. by providing them for therapeutic application.

Furthermore, 6-hydroxy-5-methylindirubin, the primary metabolite of 5-methylindirubin, surprisingly displays increased solubility in physiological saline (pH 7.4) by a factor of more than 2000: whereas the parent compound shows solubility of 0.1 mg/l, the 6-hydroxylated metabolite surprisingly dissolves up to 240 mg/l. Since the log P values (octanol/water) for parent compound and metabolite both are above 1 (1.1 as compared to 1.6) both compounds prefer lipophilic phases and easily penetrate therefore membranes. However, since the metabolite, being so much better soluble in aqueous phase, surprisingly retains high lipophilicity, its biodistribution into relevant lipophilic compartments and its ability to cross cell membranes is markedly improved. Primary ring hydroxylation thus brings about much better distribution behaviour and a considerably increased systemic bioavailability.

Another great advantage resulting from the isolation, identification and chemical synthesis of the 6-OH metabolite is its usefulness for making a specific, pharmacologically highly attractive secondary metabolite. In physiological situations, this secondary metabolite results from coupling of the primary 6-hydroxylated metabolite to glucuronic acid by glucuronyl transferases. Such glucuronated secondary metabolites, also called coupling products, in most cases are very well soluble in water and thus can be very easily excreted through the urinary tract. However, they can also be cleaved to some extent, either by glucuronidases present in the microorganismus of the gastrointestinal tract or, in anareobic parts of solid tumors. The first process can lead to enhanced absorption from the gastrointestinal tract of the primary, biologically highly active 6-OH metabolite, the second process will lead to enhanced enrichment of this metabolite in anaerobic tumor tissue since glucuronidase activity is expressed in poorly oxygenated tumor tissue, providing for local liberation of 6-OH-5-methylindirubin from the glucuronide.

The synthesis of the claimed compounds is exemplified in the following:

### (i) Synthesis of 4-methyl-3-(triisopropylsilyloxy)-aniline (2)

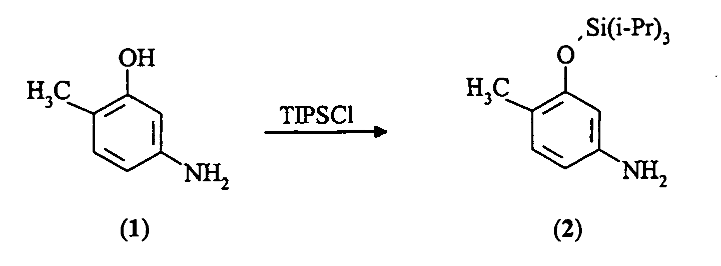

To a solution of 5-amino-2-methyl-phenol (1) (12 g, 97 mmol) and imidazole (16.3 g, 240 mmol) in 60 ml pyridine are added dropwise 25 ml (117 mmol) triisopropylsilylchloride. This mixture is stirred for 3 days at ambient temperature. After addition of water, the reaction mixture is extracted 2-3 times with 50 ml CH₂Cl₂. The CH₂Cl₂ phases are collected, washed with saturated aqueous NaCl-solution and dried over Na₂SO₄. The solvent is then stripped off. The crude reaction product (2) thus obtained (Rf = 0.48; hexane/ethyl acetate 4:1) is used for the next reaction step to the carbamate (3) without any further work-up.

### (ii) Synthesis of tert.-butyl-(4-methyl-3-triisopropylsilyloxy)-phenyl)-carbamate (3)

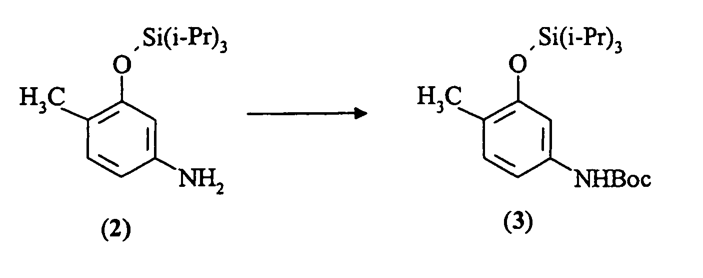

The crude reaction product (2) is dissolved in 200 ml THF and di-*tert*.-butyl dicarbonate (Boc)₂O (33 ml, 146 mmol) is added. After stirring for 20 hours at ambient temperature, the solvent is stripped off, the residue is taken up in water and extracted with CH₂Cl₂. The CH₂Cl₂ phases are collected, washed with saturated aqueous NaCl-solution and dried over Na₂SO₄. The solvent is then stripped off. Recrystallization from hexane yields pure *tert*.-butyl-(4-methyl-3-triisopropyl silyloxy)-phenyl)-carbamate (3) (Rf = 0.91; hexane/ethyl acetate 4:1) (yield: 30.1 g; 81.2 %).

### (iii) Synthesis of 6-hydroxy-5-methyl-isatin (4)

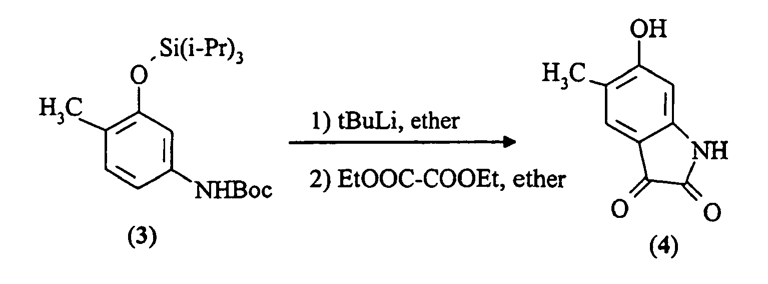

55 ml (81 mmol) of 1.5 M *tert*.-butyl lithium solution in pentane is added dropwise under argon to a solution of *tert*.-butyl-(4-methyl-3-triisopropylsilyloxy)-phenyl)-carbamate **(3)** (12.3 g, 32 mmol) in 65 ml abs. ether at -20°C. The solution is stirred for 2 hours at -20°C. Then, a solution of 5.5 ml fresh destilled diethyl oxalate (40.4 mmol) in 35 ml ether is added dropwise within 5 minutes. The cooling bath is removed after 2 hours. The reaction mixture is then stirred with 400 ml 1 N HCl and extracted with ethyl acetate. After removal of the solvent the residue is refluxed for 1 hour with 320 ml of a 1:1 mixture of ethanol and 6N HCl. After extraction, stripping off the solvent and subsequent flash column chromatography, 6-hydroxy-5-methyl-isatin **(4)** is obtained (yield: 1.1 g, 19 %).

### (iv) Synthesis of 6-hydroxy-5-methyl indirubin (6)

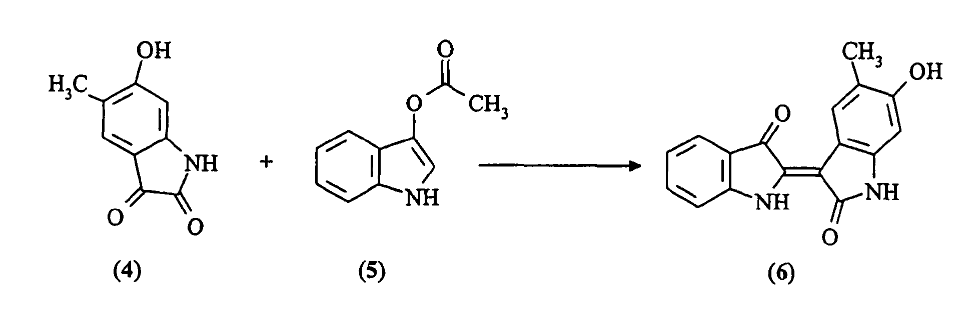

6-hydroxy-5-methyl-isatin **(4)** is dissolved in 25 ml methanol under argon. Then, indoxyl-3-acetate (5) (121 mg, 0.69 mmol) and sodium carbonate (160 mg, 1.5 mmol) are added thereto under stirring. The reaction mixture is stirred for 24 hours under ambient temperature, then extracted with ethyl acetate and water and a saturated aequeous NaCl solution as well. After work-up, 6-hydroxy-5-methyl indirubin **(6)** (Rf = 0.6; hexane/acetone 1:1) is obtained (yield: 35 mg, 15 %).
¹H-NMR-data (dmso-d₆)

| Metabolite | | | synthetized compound (**6**) | | |
|---|---|---|---|---|---|
| δ [ppm] | peak form, coupling | position | δ [ppm] | peak form, coupling | position |
| 2.11 | s | CH₃ | 2.11 | s | CH₃ |
| 6.42 | s | C7H | 6.42 | s | C7H |
| 6.96 | pt, ³J_{H,H}=7.72 Hz | C5'H | 6.96 | pt, ³J_{H,H}=7.41 Hz | C5'H |
| 7.36 | pd, ³J_{H,H}=8.02 Hz | C7'H | 7.37 | pd, ³J_{H,H}=8.18 Hz | C7'H |
| 7.52 | pt, ³J_{H,H}7.56 Hz | C6'H | 7.52 | pt, ³J_{H,H}=7.66 Hz | C6'H |
| 7.6 | pd, ³J_{H,H}=7.72 Hz | C4'H | 7.6 | pd, ³J_{H,H}=7.66 Hz | C4'H |
| 8.58 | d, ⁴J_{H,H}=0.76 Hz | C4H | 8.58 | s | C4H |
| - | - | OH | 9.97 | s | OH |
| 10.61 | s | NH or N'H | 10.62 | s | NH or N'H |
| 10.68 | s | NH or N'H | 10.69 | s | NH or N'H |

### (v) Glucuronidation of 6-hydroxy-5-methylindirubin (6)

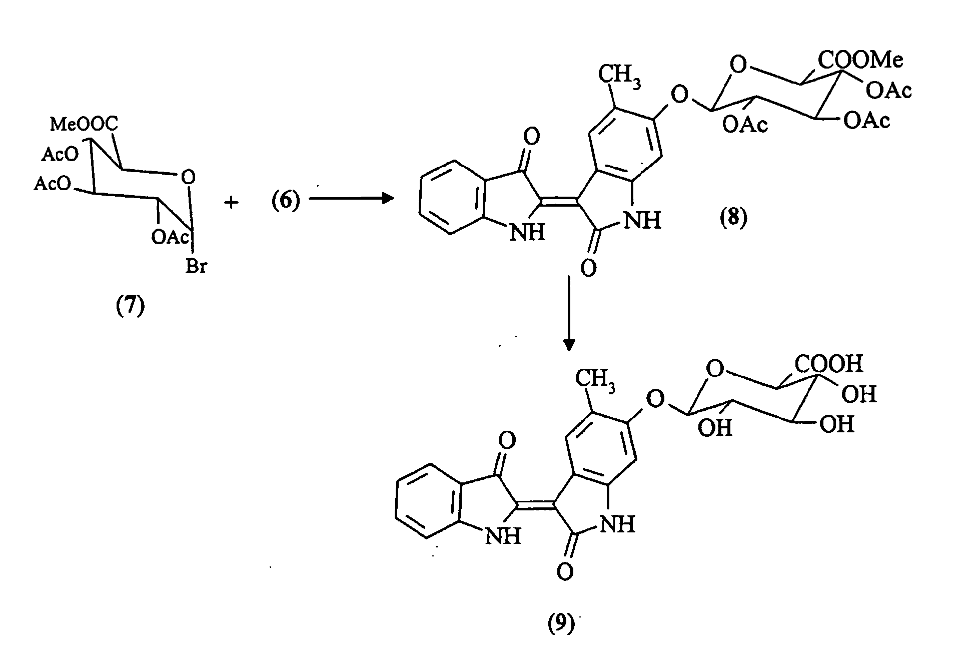

To a solution of the bromo compound **(7)** (350 mg, 0.96 mmol) in dried benzene, 4 A molecular sieve (400 mg), dried 6-hydroxy-5-methylindirubin **(6)** (340 mg, 1.92 mmol) and silver carbonate are added subsequently under inert gas. The mixture is stirred for 20 hours at ambient temperature and then filtrated. The solvent is stripped off and the crude product is purified by column chromatography (44 %). For removal of the protecting groups, compound **(8)** is stirred in a mixture of 8 ml THF, 8 ml water and 1 M NaOH (0.614 ml) at -10°C for 1 hour. After neutralization with acetic acid the solvent is stripped off and the residue is purified by column chromatography to obtain 3,4,5-trihydroxy-6-(5-methyl-1*H*,1'*H*-[2',3]bisindolyliden-2,3'-dion-6-yl)-tetrahydropyran-2-carboxylic acid **(9)** (77 %).

## Claims

1. Cell membrane penetrating indirubin derivatives having the following general formula 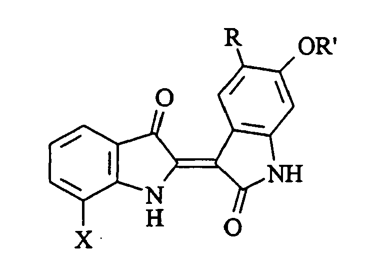 wherein the group R represents a straight-chain or branched-chain alkyl group or a straight-chain or branched-chain alkoxy group each having 1 to 18 carbon atoms, the group R' represents H, a straight-chain or branched-chain alkyl group having 1 to 4 carbon atoms or a glucuronide group, and X is H or OH.

2. Indirubin derivatives according to claim 1, wherein the group R represents a straight-chain or branched-chain alkyl group or a straight-chain or branched-chain alkoxy group each having 1 to 4 carbon atoms.

3. Indirubin derivatives according to claim 1 or 2, which are 6-hydroxy-5-methylindirubin and 6,7'-dihydroxy-5-methylindirubin and their glucuronide derivatives.

4. Indirubin derivative according to any one of the preceding claims, wherein the indirubin derivatives is in the form of a physiologically acceptable salt.

5. Pharmaceutical formulation comprising at least one of the indirubin derivatives according to any one of claims 1 to 4.

6. Use of the pharmaceutical formulation according to claim 5 for the manufacture of a medicament for the treatment of human solid tumors and leukemia and metastases thereof.

## Patentansprüche

1. Zellmembran-penetrierende Indirubinderivate mit der folgenden allgemeinen Formel 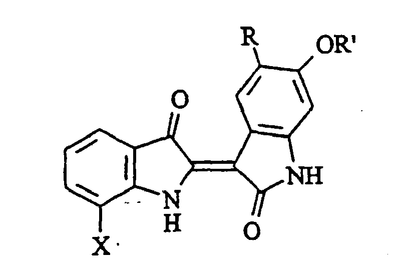 worin die Gruppe R eine geradkettige oder verzweigtkettige Alkylgruppe oder eine geradkettige oder verzweigtkettige Alkoxygruppe mit jeweils 1 bis 18 Kohlenstoffatomen darstellt, die Gruppe R' H, eine geradkettige oder verzweigtkettige Alkylgruppe mit 1 bis 4 Kohlenstoffatomen oder eine Glucuronidgruppe darstellt, und X H oder OH ist.

2. Indirubinderivate gemäß Anspruch 1, wobei die Gruppe R eine geradkettige oder verzweigtkettige Alkylgruppe oder eine geradkettige oder verzweigtkettige Alkoxygruppe mit jeweils 1 bis 4 Kohlenstoffatomen darstellt.

3. Indirubinderivate gemäß Anspruch 1 oder 2, welche 6-Hydroxy-5-methylindirubin und 6,7'-Dihydroxy-5-methylindirubin und deren Glucuronidderivate sind.

4. Indirubinderivate gemäß einem der vorhergehenden Ansprüche, wobei die Indirubinderivate in der Form eines physiologisch annehmbaren Salzes sind.

5. Pharmazeutische Formulierung, umfassend mindestens eines der Indirubinderivate gemäß einem der Ansprüche 1, bis 4.

6. Verwendung der pharmazeutischen Formulierung gemäß Anspruch 5 zur Herstellung eines Medikaments zur Behandlung von humanen soliden Tumoren und Leukämie und Metastasen davon.

## Revendications

1. Dérivés de l'indirubine pénétrant une membrane cellulaire ayant la formule générale suivante: 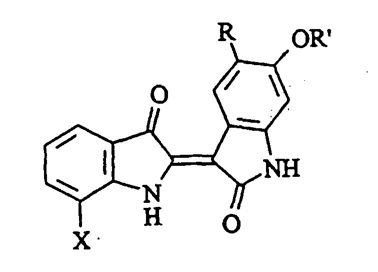 où le groupe R représente un groupe alkyle linéaire ou ramifié ou un groupe alkoxy linéaire ou ramifié, chacun ayant 1 à 18 atomes de carbone, le groupe R' représente H, un groupe alkyle linéaire ou ramifié ayant 1 à 4 atomes de carbone ou un groupe glucuronide, et X est H ou OH.

2. Dérivés de l'indirubine selon la revendication 1, où le groupe R représente un groupe alkyle linéaire ou ramifié ou un groupe alkoxy linéaire ou ramifié, chacun ayant 1 à 4 atomes de carbone.

3. Dérivés de l'indirubine selon la revendication 1 ou 2, qui sont 6-hydroxy-5-méthyl-indirubine et 6,7'-dihydroxy-5-méthyl-indirubine et leurs dérivés glucuronide.

4. Dérivés de l'indirubine selon l'une des revendications précédentes, où les dérivés de l'indirubine sont dans la forme d'un sel physiologiquement acceptable.

5. Formulation pharmaceutique comprenant au moins un des dérivés de l'indirubine selon une des revendications 1 à 4.

6. Utilisation de la formulation pharmceutique selon la revendication 5 pour la production d'un médicament pour le traitement des tumeurs solides humaines et leucémie et leurs métastases.
